# EUROPEAN PATENT SPECIFICATION

(11) **EP 0 356 239 B1**
(45) Date of publication and mention of the grant of the patent: **22.02.1995**
(21) Application number: 89308607.4
(22) Date of filing: 24.08.1989
(51) Int. Cl.: C11D 3/386

(54) **Detergent compositions**
Waschmittelzusammensetzungen
Compositions détergentes

(30) Priority: 24.08.1988 GB 8820061; 24.08.1988 GB 8820062; 19.01.1989 GB 8901182
(43) Date of publication of application: 28.02.1990
(60) Divisional application: 94112077.6
(73) Proprietor: ALLIED COLLOIDS LIMITED, Bradford, West Yorkshire BD12 0JZ (GB)
(72) Inventor: Langley, John, Shipley West Yorkshire BD17 6TM (GB); Symes, Kenneth Charles, Keighley West Yorkshire BD20 2UU (GB)
(74) Representative: Lawrence, Peter Robin Broughton

(56) References cited:
- EP-A- 0 126 528
- EP-A- 0 128 661
- EP-A- 0 351 162
- CHEMICAL ABSTRACTS, vol. 107, no. 12, September 1987 Columbus, Ohio, USA,page 111; column 2; ref. no. 98645 & JP-A-61254244
- CHEMICAL ABSTRACTS, vol. 110, no. 4, January 1989 Columbus, Ohio, USA, page 104, ref. no. 25782 & JP-A-63105098

## Description

It is well known to include enzymes in detergent compositions in order to improve their performance. The enzymes will be de-activated by exposure to other chemical components in the detergent, and possibly also upon exposure to moisture. It is therefore necessary to protect the enzyme, for instance by encapsulation within a polymer. Detergent powders therefore frequently contain powdered enzyme-polymer compositions.

A difficulty that exists with liquid detergents is that the liquid phase of the detergent concentrate is more likely to attack the encapsulating polymer and does, in any event, tend to provide a more rigorous chemical and physical environment from which the polymer has to protect the enzyme. It has proved difficult to provide the enzyme in a protected form and that is stably distributed within the liquid detergent. If the enzyme is not fully protected then the enzyme activity in the detergent, when it is used by the consumer, will be very low.

In GB-A-2,186,884 it is proposed to disperse the enzyme in a silicone oil and to disperse this dispersion in the liquid detergent. Although it is proposed that the dispersed particles can have a size as low as 2»m in practice they are always very much larger than this. This has to follow from the fact that the enzyme, that is within the dispersed particles, is stated to have a particle size that is preferably above 5»m, with the result that the dispersed particles containing the enzyme must inevitably be much larger. In practice therefore at least 90% of the particles of oil and enzyme will always have a particle size substantially above 5»m.

In JP-A-63-105098 it is proposed to microencapsulate enzyme in polyvinyl alcohol and to disperse the resultant microcapsules in a liquid or gel detergent. In practice any liquid detergents that are used would need to have a high viscosity because the microcapsules that are made are always relatively large, for instance 150 to 800»m in example 1 and so would inevitably settle out from a conventional, relatively low viscosity, liquid detergent concentrate. One way that is described for making the microcapsules involves dispersing an enzyme solution containing polyhydroxy compound and polyvinyl alcohol is dispersed in hydrophobic solvent as microdroplets and the water content is removed by heating or decrease in pressure. As mentioned, the particle size is large and this process is not exemplified.

In JP-A-61-254244 enzyme and water soluble polymer are dispersed in liquid hydrocarbon and then acetone is added. Although the use of a "culture solution" is mentioned, it is clear that the intention is to precipitate the polymer around the enzyme, rather than to form a matrix throughout which the enzyme is distributed, and for this purpose a previously dry enzyme can be used. If the resultant "shell" of polymer is damaged, all the "core" of enzyme is exposed to the environment.

The commercial reality is that there is no entirely satisfactory way of incorporating detergent enzymes into liquid detergent concentrates and, in particular, the enzymic activity of any such composition is rapidly lost during storage.

It would be desirable to be able to provide a liquid composition that can be incorporated into a liquid detergent concentrate, and it would be desirable to provide such concentrates wherein the enzymic activity can be maintained.

A liquid composition according to the invention comprises a substantially stable dispersion in a liquid phase of particles having a size below 20»m and having a substantially anhydrous core comprising a matrix polymer and a detergent enzyme distributed through the matrix polymer, and an outer shell deposited around the core of coacervated polymeric material that is impermeable to liquid detergent concentrates but which releases the enzyme when agitated in aqueous wash liquor.

Preferably the particles have been made by a process comprising forming a dispersion of an aqueous phase in a water immiscible liquid in the presence of a dispersion stabiliser wherein the aqueous phase comprises detergent enzyme and a solution or emulsion of a matrix polymer, and azeotroping the dispersion to convert the aqueous phase into particles comprising solid matrix polymer through which the detergent enzyme is distributed and the resultant particles are coated by coacervation with polymeric material.

The azeotroped product may serve as the liquid composition, in which event the liquid phase of the composition is the water immiscible liquid in which the dispersion was formed. The particles in this dispersion (and in the final product) are preferably below 10»m, most preferably below 3»m. This assists the formation of a stable dispersion.

The dispersion stabiliser that is used in this process is preferably an amphipathic polymeric stabiliser, that is to say a polymeric stabiliser having hydrophobic and hydrophilic components as a result of having been made from hydrophobic and hydrophilic monomers. Accordingly, the core particles of the dispersion each have a surrounding hydrophobic film derived from the hydrophobic units in the stabiliser and this hydrophobic film can serve as part of the protective polymeric material around the core particles. The formation of the initial dispersion, that is subjected to azeotroping, is generally facilitated by the incorporation of a water-in-oil emulsifier, and the hydrophobic groups of this may also contribute to the formation of a protective hydrophobic film around each core particle.

However it is necessary to include additional polymeric material in order to provide adequate protection. This additional material includes polymeric matrix for each particle, with the enzyme being distributed through the matrix. The matrix may be of uniform composition throughout, but preferably it is chemically modified during or after the formation of the matrix (i.e., during or after the azeotroping) so as to render it less permeable to liquid detergent concentrate. Thus the polymer may be introduced in soluble form and may then be insolubilised, particularly at the outer surface layer of the matrix.

In addition to, having a matrix polymer there is an outer polymeric shell formed by coacervation. Coacervation is usually conducted in an aqueous phase and so the liquid phase of the composition of the invention can be this coacervating liquid phase, provided the polymeric shell is sufficiently impermeable to it during storage.

The liquid compositions of the invention also include compositions wherein the liquid phase is the continuous phase of a liquid detergent concentrate. This may be formulated in conventional manner from appropriate blends of surfactants, builders and other conventional additives for liquid detergents.

The liquid detergent is normally a relatively low viscosity liquid that has a low water content and a high content of surfactant and/or electrolyte. Many polymeric materials, particularly if they are addition polymers made from a monomer or monomer blend containing ionic monomers, are much less soluble and permeable when exposed to high electrolyte or surfactant concentrations than when exposed to dilute aqueous solutions. Because of this, and because of the small particle size of the particles, it is therefore possible to provide the enzyme in a protective polymer that is substantially impermeable to the other components of the liquid detergent but which will dissolve when the water content is increased greatly, namely when the concentrate is diluted into the wash liquor for use. Although the dilution effect alone may be sufficient to release the enzyme from within the particles, reliance may also be placed on the temperature of the wash liquor, since this is generally above 30°C and usually above 40°C and so is above the normal storage temperature of the composition. In particular, reliance may be placed on agitation, for instance of the sort to which aqueous wash liquors are normally subjected, since this can promote rupture of the polymeric matrix or, especially, of any outer protective polymeric shell.

The enzyme can be any enzyme that is useful in detergents. It is preferably a protease, especially an alkaline protease, but it may for instance be an amylase or a lipase. It may be introduced initially in powder form but is generally introduced as a solution (or a dispersion containing cellular material with which it was initially produced). This solution may be formed from a dried enzyme product or it may be a fermentation liquor, for instance the fermentation broth in which the enzyme was produced initially or a concentrate obtained from that. Processes in which the enzyme is provided initially as a fermentation broth are described in EP-A-356240 published on 28.02.90.

The preferred products of the invention are made by a process that involves reverse phase azeotroping, and for this purpose an aqueous phase containing the enzyme is dispersed in a water immiscible liquid in the presence of the dispersion stabilizer. The aqueous phase should be stable both against phase separation and against loss of activity of the enzyme. For instance it can be desirable to include a polyhydroxy compound, especially sucrose or other sugar or a glycol or other low molecular weight polyhydroxy compound, e.g., propylene glycol. The dispersion of the aqueous phase into the water immiscible liquid is generally accompanied by shear and is conducted in the presence of a water-in-oil emulsifier so as to promote the formation of small particles generally having a size below 10»m, most usually below 3»m. suitable surfactants, water immiscible liquids and polymeric stabilisers, and suitable azeotroping conditions, are described in EP-A-0128661 and 0126528 and, in particular, suitable stabilisers are described in GB-A-2,002,400 with particularly preferred stabilisers being described in GB-A-2,001,083 and GB-A-1,482,515.

The immiscible liquid is non-aqueous and must include liquid that will form an azeotrope with water. Often the water immiscible liquid is a blend of a relatively high boiling liquid that remains in the dispersion and a low boiling liquid that is azeotroped from the dispersion. The temperature at which azeotroping occurs is generally below 100°C and is controlled by the choice of liquid and, especially, the pressure at which the distillation is conducted. Generally the distillation is conducted under reduced pressure and when the active ingredient is temperature sensitive (e.g., an enzyme) the reduced pressure is preferably such that the azeotroping occurs at a maximum temperature of not more than 80°C, often below 70°C and most preferably below 50°C. For instance by applying a relatively high vacuum it is possible to azeotrope at very low temperatures, for instance as low as 30°C. Sodium sulphate or other salt may be added to lower the azeotroping temperature.

The aqueous phase containing the enzyme contains a polymer so as to form a polymeric matrix through which the enzyme is distributed.

The polymer should be film forming at the distillation temperature, and usually is film forming at 20°C or lower. The azeotroping should be conducted to render the particles substantially dry so that they are not desensitised by the presence of water in them. In practice this means that the water content is generally below 25%, and preferably below 10%, by weight of the particles and most preferably is at or, especially, below the moisture content that would prevail if the particles were exposed to the atmosphere.

The polymer can be a natural or modified polymer such as a starch or a cellulose (e.g., carboxy methyl cellulose) or gum. Preferably it is a synthetic polymer formed from an ethylenically unsaturated water soluble monomer or monomer blend, which may be non-ionic or ionic.

Suitable anionic monomers are ethylenically unsaturated carboxylic or sulphonic monomers, most preferably monomers such as (meth) acrylic acid, crotonic acid, itaconic acid, maleic acid, (meth) allyl sulphonic acid, vinyl sulphonic acid and 2-acrylamido-2-methyl propane sulphonic acid. Acrylic or methacrylic acid is preferred.

Suitable cationic monomers are dialkylaminoalkyl (meth) -acrylamides and, preferably, -acrylates, usually as acid addition or quaternary ammonium salts. Particularly preferred are monomers such as diethylaminoethyl (meth) acrylate.

Suitable non-ionic monomers of this type are (meth) acrylamide and hydroxy-lower alkyl (meth) acrylates. The anionic and cationic monomers may be either in the free acid or free base form when they are sufficiently soluble in this form (for instance acrylic acid) but more usually in the form of an alkali metal or ammonium salt or anionic monomers or an acid addition or quaternary ammonium salt of cationic monomers.

The polymer may be polyvinyl pyrrolidone, polyvinyl alcohol or ethylene (meth) acrylic acid copolymer.

The preferred polymer is usually based on 0-50% acrylamide and 50-100% acrylic acid or soluble salt thereof.

The soluble polymer may have been made by any conventional polymerisation technique, such as reverse phase suspension polymerisation, solution polymerisation, reverse phase bead polymerisation or gel polymerisation. Alternatively, the polymer may be a copolymer of soluble and insoluble monomers (e.g., methacrylic acid and ethyl acrylate) and may have been made by oil-in-water emulsion polymerisation followed by addition of sodium hydroxide or other alkali to convert it to a soluble form.

Instead of introducing the polymer in a soluble form, the polymer can be a polymer that is insoluble in water but is soluble in alkali and which is introduced as an oil-in-water emulsion that has been made by emulsion polymerisation of ethylenically unsaturated monomer or monomer blend that is insoluble in the water phase of the polymerisation mixture. The monomers are generally a blend of anionic solubilising monomers (typically selected from the anionic monomers discussed above) and ethylenically unsaturated non-ionic monomers, the overall blend being insoluble at the pH of the emulsion. Thus the emulsion polymerisation may be conducted at a pH below 7 but when the polymer is subsequently exposed to more alkaline conditions the polymer becomes soluble (or highly swellable). Suitable non-ionic water insoluble monomers include alkyl (meth) acrylates, styrene, acrylonitrile, vinyl chloride, vinyl acetate or vinyl butyl ether. Ethyl acrylate is preferred, with the polymer preferably being formed from 10 to 70% methacrylic acid or other anionic monomer, 10 to 70% ethyl acrylate or other insoluble monomer and 0 to 70% acrylamide or other soluble non-ionic monomer.

The use of an emulsion polymer of this type is of particular value when it is desired for the polymeric matrix to permit substantially no release of the biological material in one environment (for instance neutral or acidic) and rapid release in an alkaline environment.

Controlled release of enzyme can also be obtained when the polymer is introduced initially as a salt with a volatile amine (for instance ammonia) of a polymer derived from ethylenically unsaturated carboxylic acid monomer such as (meth) acrylic acid. The salt is soluble in water but the ammonia or other volatile amine evaporates during the azeotroping to render the polymer less hydrophilic. Accordingly at least the outer shell of the particles, and possibly substantially the entire polymeric matrix, will be less hydrophilic and water soluble than when the carboxylic groups are in alkali or amine salt form. The particle therefore has relatively low permeability to ambient moisture but, upon exposure to a slightly alkaline aqueous solution (for instance as typically prevails in a wash liquid) the polymer will be sufficiently solubilised to permit rapid release of the trapped enzyme or other biological material. For this purpose the polymer is preferably based on 0 to 50% acrylamide and 50 to 100% acrylic acid or, preferably, methacrylic acid. Products of these types are described in more detail in our EP-A-361677 filed even date herewith and published on 04.04.90.

The matrix polymer preferably is a polymer that is useful as a component in a detergent, for instance as a detergent builder or detergent anti-redeposition aid. Suitable polymers include carboxy methyl cellulose and anionic synthetic polymers, for instance polymers of molecular weight 4,000 to 300,000 and formed from water soluble ethylenically unsaturated carboxylic or sulphonic monomer, optionally with water soluble non-ionic monomer. Preferably the polymer is of sodium polyacrylate but copolymers with arylamide and homopolymers or copolymers with, for instance, allyl sulphonate or 2-acrylamido methyl propane sulphonate may be used. Copolymers of maleic anhydride with, for instance, acrylic acid are also suitable.

It is preferred that the amount of polymer should be at least 0.5 times the amount of active ingredient (on a dry weight basis). Preferably there is an excess of polymer, for instance at least two times the amount of active ingredient and preferably there is a large excess, for instance at least seven times. The use of this seven-fold excess (or more) of polymer means that the biological material is protected very effectively from the environment and even if the particles are damaged the amount of active ingredient that is liable to be exposed to, or escape into, the environment is very low. Generally the amount of polymer is at least 10 times and usually at least 15 times the amount of active ingredient (on a dry weight basis). It is usually unnecesary for it to be more than 50 times, and amounts in the range 15 to 30 times are often suitable although amounts up to 100 times or more can be used.

Small amounts of filler and other additives can be included in the matrix. Generally the polymeric material constitutes at least 50%, preferably at least 75% and most preferably at least 90% by weight of the solid composition formed of a matrix, active ingredient and any inert material distributed through the matrix.

It is generally preferred that the aqueous phase that is to be dispersed and then azeotroped should be formed from the enzyme and preformed polymer. However if desired it can be made by polymerising the appropriate monomer or monomer blend in the presence of the enzyme so as to form the polymer in the presence of the enzyme. Alternatively, reactive polymer can be used, e.g., as in EP-A-032831 and can be reacted in the matrix or shell.

The product resulting from the azeotroping is a stable dispersion in the water immiscible liquid of substantially dry particles of which at least 90% by weight of which are preferably below 3»m, often below 2»m and frequently even below 1»m.

The polymeric particles will, in practice, generally have an absorbed layer of hydrophobic material due to emulsifier and/or polymeric stabiliser.

The matrix polymer is preferably chemically modified at least at its outer surface either during or after the azeotroping. For instance a volatile amine salt can be converted to a less volatile free acid compound or the polymer can be subjected to external chemical reaction. For instance if the polymer is formed of chitosan it can be acetylated, or otherwise esterified, so as to insolubilise it. Various other insolubilisation reactions can be conducted on water soluble polymers in known manner so as to render them less soluble or wholly insoluble in water. The necessary reagent for this reaction is preferably included in the dispersion during the azeotroping stage. For instance the surface can be subjected to cross linking.

A polymeric shell is formed by coacervation. Coacervation techniques are, of course, known for encapsulating a variety of materials and are described in, for instance, GB-A-1,275,712, 1,475,229 and 1,507,739 and DE-A-3,545,803.

Preferably a dispersion of enzyme particles in a water immiscible liquid, with the enzyme being distributed throughout a polymeric matrix, is preferably formed by azeotroping as described above, and then an emulsion of droplets of the dispersion is formed in an aqueous medium and the droplets are coated with coacervation with polymeric material while dispersed in the aqueous medium to form a coacervate dispersion. The coacervated particles are preferably stabilised against agglomeration while dispersed in the aqueous medium. This can be achieved by the addition of appropriate stabilising agents but preferably the materials used for forming the coacervate are such as to stabilise the coacervated particles against aggregation. Thus preferably the emulsification of the dispersion into the aqueous coacervating medium is conducted in the absence of any emulsifying agent, adequate initial stability be provided by the coacervating polymer or polymers in the coacervating system and final resistance against agglomeration being provided by the coacervate shell.

Although a coacervate coating can in some instances be formed by precipitation of a single polymer around the emulsified particles it is preferably formed by physical or chemical interaction between the two or more coacervating polymers in the aqueous medium into which the solution of enzyme (and usually matrix polymer) has already been dispersed, for instance to resemble an emulsion. As is known, when two coacervating polymers interact in the presence of an emulsion they tend to form a coating around the individual particles of the emulsion. However any other way of forming a stabilising polymeric coating on the particles by coacervation can be used. For instance a fine particulate coacervate can be formed in an aqueous medium in the absence of the emulsified particles and can then be contacted with the emulsified particles, either by emulsifying the organic solution into the aqueous medium containing the particulate coacervate or by blending that aqueous medium with an aqueous medium containing the emulsified particles. A method of this general type is described in DE-A-3,545,803 (except that it is not essential to react the coacervate coating subsequently with non-ionic melamine formaldehyde).

The coacervate-forming polymers that will interact to form a coacervate are generally counterionic. At least one of the polymers may be amphoteric. Particularly preferred polymers are blends of cationic formaldehyde polymer (generally cationic urea formaldehyde) and anionic acrylamide polymer. Suitable materials are described in, for instance, DE-A-3,545,803, GB-A-2,073,132 and 1,507,739 and US-A-4,100,103.

For the purposes of the present invention, it is usually sufficient merely to form the dispersion-stabilising coacervate coating around the fluid particles and it is usually unnecessary to react this further by cross linking or condensation with, for instance, further formaldehyde polymer, as is suggested in each of those patents.

Provided the emulsification of the dispersion droplets into the aqueous coacervating medium is conducted with only low amounts of shear and/or in the substantial absence of oil-in-water emulsifier, it is possible to form an emulsion of droplets of the dispersion in the aqueous medium and the eventual coacervated particles will then each consist of the outer coacervated shell, a core of one or more of the enzyme particles, comprising matrix polymer particles having enzyme distributed throughout, and an inner hydrophobic shell between these particles and the outer shell. This inner hydrophobic shell can consist solely of the original water immiscible liquid but preferably the water immiscible liquid is present in the initial dispersion that is to be azeotroped and that is encapsulated comprises a blend of a relatively volatile liquid and a less volatile hydrophobic material, and the relatively volatile liquid is evaporated from the particles during or after coacervation.

The evaporation can be conducted by distillation at atmospheric or reduced pressure and is generally conducted as an azeotropic distillation. This is generally conducted under reduced pressure. By appropriate selection of the solvent and the pressure at which distillation is conducted it is possible to effect the distillation at low temperatures, e.g., as low as 50°C or even as low as 30°C. It can be convenient to add appropriate volatile liquid and/or less volatile liquid to the azeotroped dispersion from the first stage, before the coacervation. The less volatile hydrophobic material can be either a high boiling oil or can be a solid or semi-solid material, for instance to form a wax layer around the enzyme particles.

The following are examples.

### Example 1

A 25% aqueous solution of ammonium polyacrylate having molecular weight 30,000 is blended with sufficient of a detergent alkaline proteaise to give a polymer:enzyme dry weight ration of 19:1. This solution is stirred into a paraffinic oil in the presence of a water-in-oil emulsifier and an amphipathic polymeric stabiliser using sufficient shear to form a stable emulsion in the oil of particles having a size below 3»m and consisting of the aqueous blend of polymer and enzyme.

This emulsion is then subjected to azeotropic distillation under reduced pressure such that the maximum temperature in the emulsion does not exceed about 50°C, and results in a dispersion in the oil of substantially dry particles having a size below 3»m, often below 1»m, each consisting of a matrix of water soluble polymer, mainly in the free acid form, throughout which the enzyme is uniformly distributed.

A solution of 168g of 20% aqueous acrylamide/sodium acrylate polymer is dissolved in 600g water and 76g of a 35% urea/formaldehyde resin is dissolved in 100g water and is added over a period of 20 seconds while stirring with a Silverson stirrer, stirring then being continued for a further 30 seconds. 120g of the dispersion in paraffinic oil is then stirred into this solution to form a white emulsion.

This emulsion can then be stirred into a liquid detergent concentrate.

### Example 2

The process of example 1 can be repeated except that a solution in a low boiling hydrocarbon of a waxy hydrocarbon is stirred into the azeotroped dispersion before emulsification in the coacervating polymer solution. The resultant stirred emulsion is then subjected to distillation under reduced pressure at a maximum temperature of 45°C in order to strip off most of the low boiling solvent.

## Claims

1. A liquid composition comprising a substantially stable dispersion in a liquid phase of particles comprising a detergent enzyme and polymeric material characterised in that the particles have a size below 20»m and have a substantially anhydrous core comprising a matrix polymer and a detergent enzyme distributed through the matrix polymer, and an outer shell deposited around the core of coacervated polymeric material that is impermeable to liquid detergent concentrates but which releases the enzyme when agitated in aqueous wash liquor.

2. A composition according to claim 1 in which the matrix polymeric material is an addition polymer of ethylenically unsaturated ionic monomer optionally with ethylenically unsaturated non-ionic monomer.

3. A composition according to claim 1 or claim 2 in which the particles are below 3»m in size.

4. A composition according to any one of claims 1 to 3 in which the particles have a hydrophobic material disposed between the outer shell and the core.

5. A composition according to any one of claims 1 to 4 in which the liquid phase is a liquid detergent.

6. A process for making a composition according to any preceding claim comprising forming a dispersion of an aqueous phase in a water immiscible liquid in the presence of a dispersion stabiliser wherein the aqueous phase comprises detergent enzyme and a solution or emulsion of a matrix polymer, and azeotroping the dispersion to convert the aqueous phase into particles comprising solid matrix polymer through which the detergent enzyme is distributed, and coating the resultant particles by coacervation with polymeric material.

7. A process according to claim 6 in which the matrix polymer is chemically modified during or after the azeotroping to render it less permeable to liquid detergent concentrate.

8. A process for making a composition according to any of claims 1 to 5 comprising forming a dispersion in water-immiscible liquid of the matrix polymer particles containing enzyme, forming an emulsion of droplets of the dispersion in an aqueous medium containing coacervating polymeric material, and coating the droplets by coacervation of the coacervating polymeric material while dispersed in the aqueous medium to form a coacervate dispersion of particles comprising a core of solid matrix polymer through which enzyme is distributed and an outer coacervate coating.

9. A process according to any one of claims 6, 7 or 8 in which the particles have an inner hydrophobic shell of the water immiscible liquid between the core and the outer shell.

## Patentansprüche

1. Flüssige Zusammensetzung, umfassend eine im wesentlichen stabile Dispersion in einer flüssigen Phase von Teilchen, die ein Detergens-Enzym und polymeres Material umfassen, dadurch gekennzeichnet, daß die Teilchen eine Größe unter 20 »m besitzen und einen im wesentlichen wasserfreien Kern, der ein Matrix-Polymer und ein Detergens-Enzym, das innerhalb des gesamten Matrix-Polymers verteilt ist, umfaßt, und eine um den Kern herum abgeschiedene äußere Schale aus coazerviertem polymeren Material, das gegenüber flüssigen Detergens-Konzentraten undurchlässig ist, aber beim Rühren in wäßriger Waschlauge Enzym freigibt, aufweisen.

2. Zusammensetzung nach Anspruch 1, in der das polymere Matrix-Material ein Additionspolymer von ethylenisch ungesättigtem ionischem Monomer, gegebenenfalls mit ethylenisch ungesättigtem nicht-ionischem Monomer, ist.

3. Zusammensetzung nach Anspruch 1 oder Anspruch 2, in der die Teilchen eine Größe unter 3 »m aufweisen.

4. Zusammensetzung nach irgendeinem der Ansprüche 1 bis 3, in der die Teilchen in hydrophobes Material, das zwischen der äußeren Schale und dem Kern angeordnet ist, aufweisen.

5. Zusammensetzung nach irgendeinem der Ansprüche 1 bis 4, in der die flüssige Phase ein flüssiges Detergens ist.

6. Verfahren zur Herstellung einer Zusammensetzung gemäß irgendeinem vorangehenden Anspruch, umfassend das Bilden einer Dispersion einer wäßrigen Phase in einer mit Wasser nicht mischbaren Flüssigkeit in Anwesenheit eines Dispersions-Stabilisators, worin die wäßrige Phase Detergens-Enzym und eine Lösung oder Emulsion eines Matrix-Polymers umfaßt, und die Azeotropisierung der Dispersion, um die wäßrige Phase in Teilchen umzuwandeln, die festes Matrix-Polymer, in dem das Detergens-Enzym durchgehend verteilt ist, umfaßt, umzuwandeln, und das Beschichten der resultierenden Teilchen durch Coazervation mit polymerem Material.

7. Verfahren nach Anspruch 6, in welchem das Matrix-Polymer während oder nach der Azeotropisierung chemisch modifiziert wird, um es gegenüber flüssigem Detergens-Konzentrat weniger durchlässig zu machen.

8. Verfahren zur Herstellung einer Zusammensetzung nach irgendeinem der Ansprüche 1 bis 5, umfassend das Bilden einer Dispersion in mit Wasser nicht mischbarer Flüssigkeit der Enzym enthaltenden Matrix-Polymer-Teilchen, das Bilden einer Emulsion von Tröpfchen der Dispersion in einem wäßrigen Medium, das coazervierendes polymeres Material enthält, und das Beschichten der Tröpfchen durch Coazervation des coazervierenden polymeren Materials, während sie in dem wäßrigen Medium dispergiert sind, um eine Coazervat-Dispersion von Teilchen zu bilden, die einen Kern aus festem Matrix-Polymer, in dem Enzym durchgehend verteilt ist, und einen äußeren Coazervat-Überzug umfassen.

9. Verfahren nach irgendeinem der Ansprüche 6, 7 oder 8, in dem die Teilchen eine innere hydrophobe Schale aus der mit Wasser nicht mischbaren Flüssigkeit zwischen dem Kern und der äußeren Schale aufweisen.

## Revendications

1. Composition liquide comprenant une dispersion pratiquement stable dans une phase liquide de particules contenant une enzyme détergente et un produit polymère, caractérisée en ce que les particules ont une dimension inférieure à 20 »m et présentent un coeur pratiquement anhydre comprenant un polymère matriciel et une enzyme détergente distribuée dans le polymère matriciel, ainsi qu'une enveloppe externe déposée autour du coeur du produit polymère coacervé qui est imperméable à des concentrés détergents liquides mais qui libère l'enzyme lors d'une agitation en solution aqueuse de lavage.

2. Composition selon la revendication 1, dans laquelle le produit polymère matriciel est un polymère d'addition d'un monomère ionique éthyléniquement insaturé et éventuellement d'un monomère non ionique éthyléniquement insaturé.

3. Composition selon la revendication 1 ou la revendication 2, dans laquelle les particules ont une dimension inférieure à 3 »m.

4. Composition selon l'une quelconque des revendications 1 à 3, dans laquelle les particules présentent une substance hydrophobe placée entre l'enveloppe externe et le coeur.

5. Composition selon l'une quelconque des revendications 1 à 4, dans laquelle la phase liquide est un détergent liquide.

6. Procédé de préparation d'une composition selon l'une quelconque des revendications précédentes, comprenant la formation d'une dispersion d'une phase aqueuse dans un liquide immiscible à l'eau en présence d'un stabilisant de dispersion, pour laquelle la phase aqueuse comprend une enzyme détergente et une solution ou émulsion d'un polymère matriciel, ainsi que la formation de la dispersion en azéotrope pour transformer la phase aqueuse en particules contenant un polymère solide matriciel dans lequel est distribuée l'enzyme détergente, et le revêtement des particules résultantes par coacervation avec le produit polymère.

7. Procédé selon la revendication 6, dans lequel on modifie chimiquement le polymère matriciel pendant ou après la formation de l'azéotrope pour rendre le polymère moins perméable à un concentré détergent liquide.

8. Procédé de préparation d'une composition selon l'une quelconque des revendications 1 à 5, comprenant la formation d'une dispersion dans un liquide immiscible à l'eau des particules de polymère matriciel contenant l'enzyme, la formation d'une émulsion de gouttelettes de la dispersion dans un milieu aqueux contenant un produit polymère de coacervation et le revêtement des gouttelettes par coacervation du produit polymère de coacervation tandis qu'il est dispersé dans le milieu aqueux pour former une dispersion de coacervat des particules contenant un coeur de polymère solide matriciel, dans lequel l'enzyme est distribuée ainsi qu'un revêtement de coacervat externe.

9. Procédé selon l'une quelconque des revendications 6, 7 ou 8, dans lequel les particules présentent une enveloppe interne hydrophobe du liquide immiscible à l'eau entre le coeur et l'enveloppe externe.
